(19) Europäisches Patentamt
European Patent Office
Office européen des brevets

(11) Veröffentlichungsnummer: **0 010 183**
**B1**

(12) # EUROPÄISCHE PATENTSCHRIFT

(45) Veröffentlichungstag der Patentschrift: **16.06.82**

(51) Int. Cl.³: **C 07 C 49/167,** C 07 C 45/63

(21) Anmeldenummer: **79103589.2**

(22) Anmeldetag: **24.09.79**

(54) **3,3-Bisfluoromethyl-butan-2-on und Verfahren zu dessen Herstellung.**

(30) Priorität: **06.10.78 DE 2843767**
**10.05.79 DE 2918895**

(43) Veröffentlichungstag der Anmeldung: **30.04.80 Patentblatt 80/9**

(45) Bekanntmachung des Hinweises auf die Patenterteilung: **16.06.82 Patentblatt 82/24**

(84) Benannte Vertragsstaaten: **BE CH DE FR GB IT NL**

(56) Entgegenhaltungen:
**EP-A-0 005 249**
**DE-C-710 129**

**SCIENCE PROGRESS, Vol. 58, 1970, Oxford, GB, R.G. PLEVEY et al.: «The chemistry of organofluorine compounds», Seiten 482–506**

(73) Patentinhaber: **BAYER AG, Zentralbereich Patente, Marken und Lizenzen, D-5090 Leverkusen 1, Bayerwerk (DE)**

(72) Erfinder: **Jautelat, Manfred, Dr., Müllersbaum 28, D-5093 Burscheid 1 (DE)**
Erfinder: **Stetter, Jörg, Dr., Pahlkestrasse 3, D-5600 Wuppertal 1 (DE)**
Erfinder: **Arlt, Dieter, Dr., Rybniker Strasse 2, D-5000 Köln 80 (DE)**
Erfinder: **Krämer, Wolfgang, Dr., Am Eckbusch 39/57, D-5600 Wuppertal 1 (DE)**

**EP 0 010 183 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 3,3-Bisfluormethyl-butan-2-on und Verfahren zu dessen Herstellung

Die vorliegende Erfindung betrifft das neue 3,3-Bisfluormethyl-butan-2-on, welches als Zwischenprodukt für die Synthese von Pflanzenschutzmitteln verwendet werden kann, und ein Verfahren zu dessen Herstellung.

Es ist bereits bekanntgeworden, dass bestimmte Halogenderivate des 3,3-Dimethyl-butan-2-ons interessante Zwischenprodukte zur Herstellung von Pflanzenschutzmitteln sind; als Beispiele zu nennen sind hier das 3,3-Dimethyl-4-chlor-butan-2-on und das 3,3-Dimethyl-4-brom-butan-2-on, die zu Endprodukten mit fungizider Wirksamkeit (vgl. DE-OS 2 632 602 [Le A 17 274] und DE-OS 2 632 603 [Le A 17 273]) und mit antimikrobieller Wirksamkeit (vgl. DE-OS 2 632 601 [Le A 17 275]) weiter verarbeitet werden können.

Weiterhin ist in einer vorgängigen Patentanmeldung das 3,3-Dimethyl-4-fluor-butan-2-on als Ausgangsprodukt genannt (vgl. EP-A 0 005 249). Das 3,3-Bisfluormethyl-butan-2-on und dessen Verwendung als interessantes Zwischenprodukt sind jedoch bisher noch nicht bekanntgeworden.

Es wurde nun das neue 3,3-Bisfluormethyl-butan-2-on der Formel

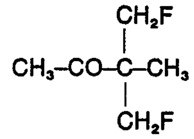

(I)

gefunden.

Man erhält die Verbindung der Formel I, wenn man den Sulfonsäureester der Formel

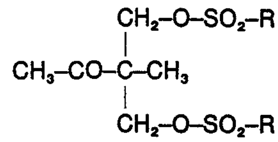

(II)

in welcher

R für Alkyl- oder Aryl-Reste steht,

mit Metallfluoriden im Temperaturbereich zwischen 80 und 250 °C in einem Verdünnungsmittel umsetzt.

Die neue Verbindung ist ein interessantes Zwischenprodukt zur Herstellung von Pflanzenschutz-Wirkstoffen mit fungizider Wirkung.

Verwendet man zur Herstellung der Verbindung 2-Acetyl-2-methyl-propan-1,3-diol-bis-methansulfonat und Kaliumfluorid als Ausgangsstoffe, so kann der Reaktionsablauf durch das folgende Formelschema wiedergegeben werden:

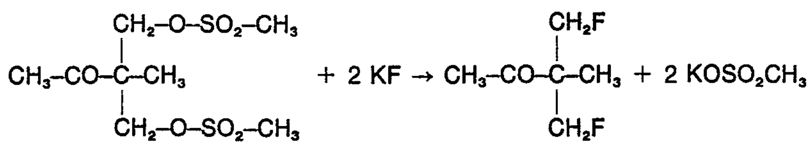

In der Formel II steht R vorzugsweise für Alkyl mit 1 bis 4 Kohlenstoffatomen, wie zum Beispiel Methyl, oder Aryl mit 6 bis 12 Kohlenstoffatomen, wie zum Beispiel Phenyl und Tolyl. Als Beispiel für einen Sulfonsäureester der Formel II sei genannt:

2-Acetyl-2-methyl-propan-1,3-diol-bis-methansulfonat.

Die als Ausgangsstoffe verwendeten Sulfonsäureester der Formel (II) sind teilweise bekannt [J. Org. Chem. 35, 2391 (1970)]. Die noch nicht beschriebenen Verbindungen können nach literaturbekannten Verfahren aus den entsprechenden Hydroxy-butanonen und Sulfochloriden in Gegenwart von Basen hergestellt werden (siehe z.B. Houben-Weyl, Methoden der Org. Chemie, Bd. IX, S. 388 und 663, sowie die Angaben bei den Herstellungsbeispielen).

Als Metallfluoride werden Alkali- und Erdalkalifluoride, vorzugsweise Natriumfluorid und Kaliumfluorid verwendet. Diese Verbindungen sind allgemein bekannt.

Als Verdünnungsmittel kommen alle polaren organischen Lösungsmittel in Frage. Hierzu gehören vorzugsweise Sulfolan, Dimethylformamid, Dimethylsulfoxid, Hexamethylphosphorsäuretriamid, Ethylenglykol, Diethylenglykol, Triethylenglykol, Tetraethylenglykol und höher kondensierte Polyäther.

Die Reaktionstemperaturen können in einem grösseren Bereich variiert werden. Im allgemeinen arbeitet man zwischen 80 und 250 °C, vorzugsweise zwischen 100 und 200 °C.

Die Herstellung der erfindungsgemässen Verbindungen kann sowohl unter Atmosphärendruck als auch unter vermindertem Druck erfolgen. Nach einer besonderen Ausführungsform führt man die Umsetzung bei einem Druck von 10 bis 100 mbar durch und destilliert das entstandene Reaktionsprodukt sogleich aus der Reaktionsmischung aus.

Bei der Durchführung dieses Verfahrens setzt man die Reaktionskomponenten Sulfonsäureester und Metallfluorid im Molverhältnis 1:1 bis 1:10, vorzugsweise 1:1,5 bis 1:3 ein.

Wie schon erwähnt, stellt die neue Verbindung der Formel I ein interessantes Zwischenprodukt dar. Sie lässt sich leicht in ein Halogenketon der Formel

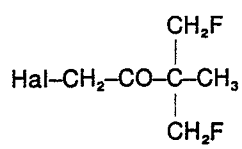

(III)

in welcher

Hal für Chlor oder Brom steht,

überführen, indem man die Verbindung der Formel I in einem inerten organischen Lösungsmittel bei Raumtemperatur mit Chlor oder Brom versetzt, oder z.B. mit üblichen Chlorierungsmitteln, wie Sulfurylchlorid, bei 20 bis 60 °C umsetzt (vgl. auch die Herstellungsbeispiele).

Die Halogenketone der Formel (III) lassen sich weiterhin mit Phenolen umsetzen (sogenannte «Williamsonsche Äthersynthese», vgl. dazu DE-OS 2 632 603 [Le A 17 273]), wobei man zu Verbindungen der Formel

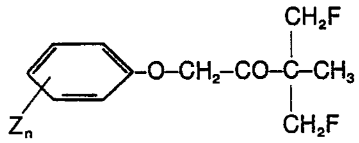
(IV)

in welcher

Z für Halogen, Alkyl, Nitro, Cyano, Alkoxycarbonyl oder gegebenenfalls substituiertes Phenyl steht und

n für 0, 1, 2 oder steht,

gelangt. Die Verbindungen der Formel (IV) können durch weitere Halogenierung, vorzugsweise mit Sulfurylchlorid oder mit Brom, in fluorierte 1-Chlor- oder 1-Brom-1-phenoxy-3,3-dimethyl-butan-2-one überführt werden, die sich mit Azolen glatt umsetzen lassen; z.B. erhält man mit 1,2,4-Triazol Verbindungen der allgemeinen Formel:

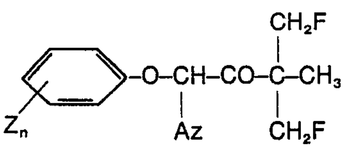
(V)

in welcher

Z und n die weiter oben angegebene Bedeutung besitzen und

Az für 1,2,4-Triazol-1-yl oder 1,2,4-Triazol-4-yl steht.

Die Verbindungen der Formel (V) weisen starke fungizide Eigenschaften auf und sind daher als Pflanzenschutzmittel verwendbar.

Herstellungsbeispiel

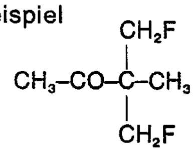

In einem Dreihalskolben mit Rührer, Tropftrichter und Liebigkühler mit gekühlter Vorlage werden 400 ml Tetraethylenglykol und 46,4 g Kaliumfluorid (0,8 Mol) vorgelegt und auf 170 °C aufgeheizt. Man legt an den Vorstoss des Liebigkühlers ein Wasserstrahlvakuum (Druck etwa 20 bis 30 mbar) an. Dann werden während 45 Minuten 57,6 g (0,2 Mol) 2-Acetyl-2-methyl-propan-1,3-diol-bismethansulfat, gelöst in 100 ml Tetraäthylenglykol, zugetropft. Das entstehende 3,3-Bisfluormethyl-butan-2-on wird während der Reaktion in die gekühlte Vorlage abdestilliert. Nach dem Zutropfen wird noch während 1 Stunde bei 175 °C weiter destilliert.

Das aufgefangene Destillat wird anschliessend redestilliert. Man erhält 14 g (etwa 51,5% der Theorie) 3,3-Bisfluormethyl-butan-2-on vom Kp. 16 mbar/43–46 °C.

Vorprodukt:

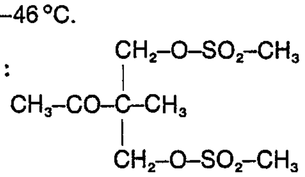

66 g (0,5 Mol) 3-Oxo-2,2-bis-[hydroxymethyl]-butan (zur Herstellung vgl. Beilstein H 1, E III 3306, IV 4132 und J. Chem. Soc., London, 1932, 2671) werden in 300 ml 1,2-Dichlorethan gelöst, 114,5 g (1 Mol) Methansulfonsäurechlorid zugetropft und bei 0 bis 5 °C 158 g (2 Mol) Pyridin zugetropft. Man lässt 15 Stunden bei Raumtemperatur nachrühren und gibt dann den Ansatz auf 600 ml Eiswasser und 100 ml konz. Salzsäure. Dabei fällt ein Feststoff aus, der abgesaugt wird. Die wässrige Phase wird mit 1 l Methylenchlorid extrahiert; in der Methylenchloridphase wird der Feststoff gelöst, die organische Phase über Natriumsulfat getrocknet, das Lösungsmittel im Wasserstrahlvakuum abdestilliert und der Rückstand in 200 ml Äther suspendiert. Der Rückstand wird abgesaugt und mit 100 ml Äther gewaschen. Man erhält 100 g (etwa 70% der Theorie) 2-Acetyl-2-methylpropan-1,3-diol-bis-methansulfonat vom Schmelzpunkt 105 bis 108 °C.

**Patentansprüche**

1. 3,3-Bisfluormethyl-butan-2-on der Formel

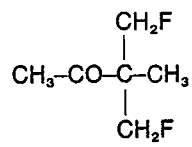
(I)

2. Verfahren zur Herstellung des 3,3-Bisfluormethyl-butan-2-ons, dadurch gekennzeichnet, dass man einen Sulfonsäureester der Formel

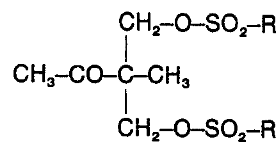
(II)

in welcher

R für Alkyl- oder Aryl-Reste steht,

mit Metallfluoriden im Temperaturbereich zwischen 80 und 250 °C in einem Verdünnungsmittel umsetzt.

3. Verfahren nach Anspruch 2, dadurch gekennzeichnet, dass man als Metallfluoride Fluoride von Metallen der 1. oder 2. Hauptgruppe des Periodensystems der Elemente verwendet.

**Claims**

1. 3,3-Bisfluoromethyl-butan-2-one of the formula

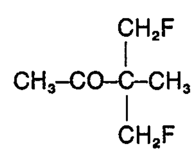
(I)

2. Process for the preparation of 3,3-bisfluoromethyl-butan-2-one, characterised in that a sulphonic acid ester of the formula

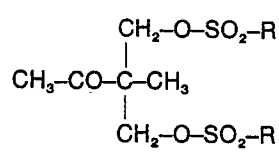
(II)

in which

R represents alkyl or aryl radicals is reacted with metal fluorides in the temperature range between 80 and 250 °C, in a diluent.

3. Process according to Claim 2, characterised in that the metal fluorides used are the fluorides of metals of main groups 1 or 2 of the periodic table of the elements.

**Revendications**

1. La 3,3-bisfluorométhyl-butane-2-one de formule:

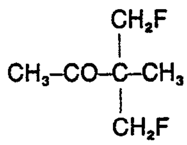

(I)

2. Procédé de production de la 3,3-bisfluorométhyl-butane-2-one, caractérisé en ce qu'on fait réagir un ester d'acide sulfonique de formule:

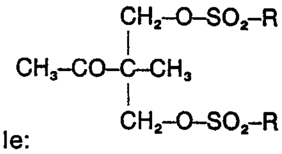

(II)

dans laquelle:

R désigne des restes alkyle ou aryle,

avec des fluorures métalliques dans la plage de températures de 80 à 250 °C dans un diluant.

3. Procédé suivant la revendication 2, caractérisé en ce qu'on utilise comme fluorures métalliques des fluorures de métaux du premier ou du deuxième groupe principal du Système Périodique des Eléments.